# EUROPÄISCHE PATENTSCHRIFT

(11) **EP 1 234 816 B1**
(45) Veröffentlichungstag und Bekanntmachung des Hinweises auf die Patenterteilung: **30.06.2004**
(21) Anmeldenummer: 01104399.9
(22) Anmeldetag: 23.02.2001
(51) Int. Cl.: C07C 233/31, A61K 31/164, A61P 31/18

(54) **Scyphostatin-Analoga als SMase-Inhibitoren**
Scyphostatin analogues as SMase inhibitors
Analogues de scyphostatin comme inhibiteurs de SMase

(43) Veröffentlichungstag der Anmeldung: 28.08.2002
(73) Patentinhaber: Biofrontera Pharmaceuticals GmbH, 51377 Leverkusen (DE)
(72) Erfinder: Deigner, Hans-Peter, Dr., 69514 Laudenbach (DE); Kinscherf, Ralf, Dr., 69198 Schriesheim (DE); Claus, Ralf A., Dr., 69190 Walldorf (DE)
(74) Vertreter: Schüssler, Andrea, Dr.

(56) Entgegenhaltungen:
- PATENT ABSTRACTS OF JAPAN vol. 1996, no. 01, 31. Januar 1996 (1996-01-31) & JP 07 233158 A (SANKYO), 5. September 1995 (1995-09-05)
- F. NARA, ET AL.: "Biological activities of scyphostatin, a neutral sphingomyelinase inhibitor from a discomycete, Trichopeziza mollissima" JOURNAL OF ANTIBIOTICS, Bd. 52, Nr. 6, Juni 1999 (1999-06), Seiten 531-535, XP002171669 Japan Antibiotics Research Association, Tokyo, JP ISSN: 0021-8820
- F. NARA, ET AL.: "Scyphostatin, a neutral sphingomyelinase inhibitor from a discomycete, Trichopeziza mollissima: taxonomy of the producing organism, fermentation, isolation, and physico-chemical properties" JOURNAL OF ANTIBIOTICS, Bd. 52, Nr. 6, Juni 1999 (1999-06), Seiten 525-530, XP002171670 Japan Antibiotics Research Association, Tokyo, JP ISSN: 0021-8820

## Beschreibung

Die vorliegende Erfindung betrifft neue Scyphostatin-Analoga, die sich dadurch auszeichnen, daß sie als Inhibitoren von SMase wirken und somit von therapeutischem Nutzen bei einer Reihe von Erkrankungen sind wie z.B. einer HIV-Infektion, neurodegenerativen Erkrankungen, inflammatorischen Erkrankungen, Ischämie, Myokardinfarkt, Schlaganfall oder bei Atherosklerose.

Sphingomyelin (*N*-acyl-sphingosyl-phosphoryl-cholin, SM) ist ein integraler Baustein sowohl biologischer Membranen als auch von Plasma-Lipoproteinen und eine wichtige Quelle zur Entstehung von Botenstoffen ("second messenger") wie Ceramid, das nach Stimulation durch Enzyme freigesetzt wird ("Sphingomyelin-pathway"). Diese Signalübertragung durch Membransphingolipide wurde in den letzten Jahren intensiv untersucht. SM wird durch Isoformen eines der Phospholipase-C ähnlichen Enzyms, genannt Sphingomyelinase (SMase), metabolisiert. Bis heute sind fünf Isotypen der SMase bekannt. Die meisten Säugetierzellen sind in der Lage, Signale über den SM-Weg zu übertragen, wobei die Signalübertragung sowohl durch Rezeptoren wie zum Beispiel Tumor-Nekrose-Faktor (TNF)-Rezeptoren bzw. Interleukin-1-Rezeptoren als auch durch Streß wie z.B. UV-Licht, Oxidantien oder Strahlung aktiviert wird. Die Bildung von Ceramid wird heute als mit ausschlaggebend für die Einleitung lebenswichtiger Zellprozesse wie zum Beispiel Zellproliferation und Apoptose angesehen. Dieser Zusammenhang zwischen Ceramid-Bildung und Induktion der Apoptose beinhaltet eine Aktivierung jeweils bestimmter Isoformen der SMase, die in saurem (saure SMasen; acid SMases, aSMasen) bzw. in neutralem Milieu (neutrale SMasen; nSMasen) ihr pH-Optimum besitzen.

Apoptose, der programmierte Zelltod, verläuft in einer unumkehrbaren Sequenz morphologischer und biochemischer Veränderungen. Sie ist der wichtigste regulative Mechanismus zur Eliminierung nicht mehr benötigter Zellen während der embryonalen Entwicklung und des Wachstums oder irreparabel geschädigter Zellen. Verschiedene Noxen wie TNF/NGF, Hitzeschock, Zytostatika, ionisierende Strahlung, Infektionen durch Viren oder Bakterien, Entzug verschiedener Wachstumsfaktoren sowie zellmembranpermeable Ceramide induzieren Apoptose in unterschiedlichen Zelltypen. Ceramid hat als "second messenger" in verschiedensten Geweben Anteil am programmierten Zelltod. Es ist z.B. über eine Ceramid-aktivierte Protein-Kinase an der Regulierung der Signal-Transduktion beteiligt, was schließlich zu Apoptose führt.

Der Ceramid-induzierte Zelltod spielt möglicherweise eine Rolle bei verschiedensten Krankheiten wie Sepsis, Atherosklerose, neurodegenerativen Krankheiten wie z.B. Morbus Alzheimer, Morbus Parkinson und Infektionen durch Retroviren wie z.B. dem HIV-Virus, wobei jeweils von einer Ceramid-vermittelten Apoptose auszugehen ist.

So resultiert eine Infektion mit dem HIV-Virus, Typ 1 (HIV-1) zusammen mit Veränderungen im zellularen Metabolismus im Verlust von Subpopulationen von T-Lymphozyten, insbesondere der CD4⁺ Helferzellen. Es gibt zunehmend Belege dafür, daß das intrazelluläre Redoxsystem, d.h. das Gleichgewicht zwischen Oxidantien und Antioxidantien, HIV-Infektion und Lymphozyten-assoziiertes Ceramid über die Aktivierung des SM-Weges verbunden sind. An der HIV-Infektion ist auch das Ceramid-Signalsystem entweder bei der Regulierung der HIV-Expression oder bei der Induktion von Apoptose beteiligt. Es konnte gezeigt werden, daß Ceramid die Signalkaskade initiiert, die zur Apoptose von Lymphozyten bei HIVinfizierten Patienten führt. Dies wird als einer der Hauptursachen bei der Abnahme von T-Lymphozyten angesehen, was schließlich zur Ausprägung des vollständigen Krankheitsbildes führt. Dies geschieht durch Induktion der Apoptose durch das CD95/APO-1/Fas-Rezeptor/Liganden-System, das die aSMase aktiviert und zur Ceramid-Produktion führt. Wird dieses durch Behandlung mit L-Carnitin inhibiert, so wird sowohl *in vitro* als auch *in vivo* die Ceramid-Synthese verhindert. Letztlich scheint der Zustand der Redox-Systeme und der Fortschritt des Ceramid-Metabolismus Voraussagen über die Intensität der Infektion zuzulassen.

Apoptose spielt auch eine große Rolle beim neuronalen Zelltod. Man vermutet eine direkte Verbindung zwischen oxidativem Streß und dem Auftreten neurodegenerativer Krankheiten. Der Zelltod bei neurodegenerativen Krankheiten, der durch oxidativen Streß verursacht werden kann, läßt sich auf intrazelluläre Signale zur SMase-Aktivierung und daraus folgender Ceramid-Produktion zurückführen. Oxidativem Streß wird zum Beispiel bei der Auslösung von Morbus Parkinson und Morbus Alzheimer eine große Rolle zugeschrieben. Die Mechanismen, die diese Krankheiten induzieren, sind zwar noch nicht völlig geklärt, aber es läßt sich bei den Erkrankten in den betroffenen Hirnarealen eine erhöhte Apoptoserate feststellen, die vermutlich auf eine größere Ceramid-Konzentration zurückzuführen ist.

SM/Ceramid ist auch bei chronischen oder akuten inflammatorischen Erkrankungen involviert. Sowohl Sepsis und die Folgen des septischen Schocks als auch Multiorganversagen haben sich zur Haupttodesursache in den Intensivstationen entwickelt. Basierend auf klinischen, experimentellen und epidemiologischen Daten wird Sepsis heute als eine inflammatorische Antwort auf Infektionen mit Anzeichen schwerer Organ-Fehlfunktionen angesehen. Dabei ist TNFα der zentrale Mediator bei der Entwicklung des septischen Schocks. Bei septischen Patienten sind Serum-TNFα-Konzentrationen und auch zellassoziierte Ceramid-Konzentrationen erhöht. Es konnte gezeigt werden, daß eine direkte Verbindung zwischen Ceramid und TNFα-Konzentrationen besteht. Da TNFα eine intrazelluläre Ceramid-Bildung hervorruft, könnten trotz erhöhter TNFα-Konzentrationen im Plasma die durch TNFα fehlregulierte inflammatorische Antwort durch Zugabe von SMase-Inhibitoren blockiert werden, was die Ceramid-Bildung vermindern würde. Qualitativ ähnliche Beobachtungen konnten auch bei anderen, Sepsis auslösenden, zellulären Reaktionen gemacht werden. Durch einen Eingriff in den Sphingolipid-Metabolismus und eine damit gesteuerte bzw. verringerte Ceramid-Produktion wären Möglichkeiten zur Kontrolle der Apoptoserate und damit der Behandlung septischer Patienten gegeben.

Schließlich spielen SM-Metaboliten und SMase auch bei der Atherogenese eine Rolle. Der (Phospho-)Lipid-Protein-Komplex "low density lipoprotein" (LDL) ist wichtig für Phosphound neutrale Lipide im Plasma und sehr anfällig für oxidative und enzymatische Modifikation. Die oxidative oder enzymatische Modifikation von LDL beinhaltet eine Vielzahl chemischer und biophysikalischer Änderungen des Lipoproteins. Das daraus resultierende modifizierte LDL (mLDL) wird als das biologisch relevante proatherogene Lipoprotein angesehen, das eine Fülle aktiver Komponenten enthält. Zelluläre Fehlregulationen oder die Verletzung von Endothelzellen durch Aktivierung des zellulären Selbstmordmechanismus, der zur Apoptose führt, werden als die Schlüsselschritte bei der Entwicklung atherosklerotischer Plaques betrachtet. Es wurde festgestellt, daß mLDL eine doppelte Rolle bei Atherosklerose spielt: niedrige Dosen von mLDL sind an der Proliferation und Synthese der extrazellulären Matrix der glatten Muskelzellen (SMC, smooth muscle cells) während des Frühstadiums der Atherosklerose beteiligt. Im fortgeschrittenen Stadium andererseits, wenn reichlich mLDL im Plaque ist, kann es Apoptose in den SMC induzieren.

Dies führt zur Instabilität und zu Rupturen der Plaques und somit zu den klinischen Folgen der Artherosklerose. mLDL induziert die Proliferation und Apoptose der SMC. Obwohl hierbei viele Mechanismen nicht geklärt sind, scheint Ceramid als ein Effektor einer durch mmLDL hervorgerufenen zellulären Antwort zu wirken. mmLDL ist ein Oxidationsprodukt von LDL (mmLDL, minimally modified LDL). mmLDL, das selbst einen beträchtlichen Anteil von SM enthält, liefert nach Internalisation und lysosomaler Degradation Substrat zur Ceramid-Bildung und stimuliert den SM/Ceramid-Weg. Die Reduktion der aSMase-Aktivität bietet eine Möglichkeit, den mLDL-vermittelten Zelltod zu modulieren.

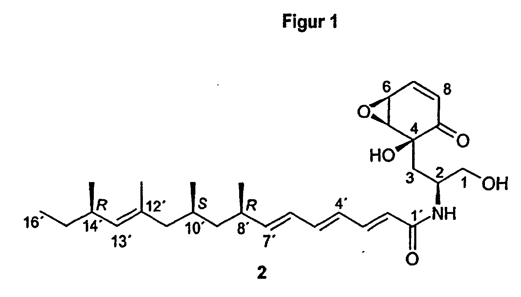

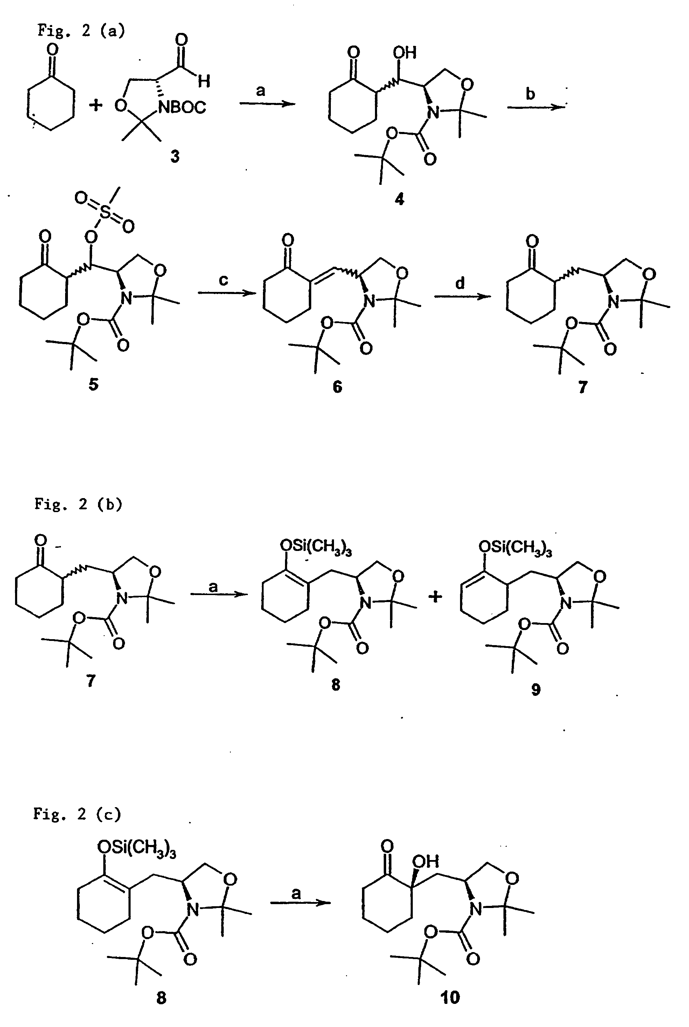

Bei der Entwicklung neuer (präventiver) therapeutischer Ansätze stellt folglich die Modulation der endogenen Ceramidgeneration, vor allem deren Reduktion, einen zentralen Aspekt dar. Dies kann z.B. durch Hemmung der membrangebundenen neutralen Sphingomylinase (N-Smase) erfolgen. Ein natürlicher Inhibitor der Sphingomylinase ist bekannt. Dabei handelt es sich um Scyphostatin (Figur 1), das erstmals 1997 aus *Trichopeziza mollissima* isoliert werden konnte^{1,2}. Allerdings ist dieser Inhibitor bisher medizinisch kaum einsetzbar, da er chemisch und metabolisch sehr instabil ist. Außerdem bestand bisher keine Möglichkeit, diesen Inhibitor oder Analoge davon synthetisch zu erhalten.

Somit liegt der Erfindung im wesentlichen das technische Problem zugrunde, neue Scyphostatin-Analoga bereitzustellen, die die Nachteile des natürlich vorkommenden Scyphostatin nicht aufweisen, d.h. synthetisch hergestellt werden können und eine erhöhte chemische und metabolische Stabilität aufweisen.

Die Lösung dieses technischen Problems wurde durch die Bereitstellung der in den Patentansprüchen gekennzeichneten Ausführungsformen erreicht.

Die vorliegende Erfindung beinhaltet erstmalig die chemische Synthese biologisch wirksamer Analoga von Scyphostatin. Durch Variation der beschriebenen Stufen ist eine Wirkstoffoptimierung erzielbar. Gegenüber dem Fermentationsverfahren des Naturstoffs ist beispielsweise die Substitution der Fettsäureamidseitenkette möglich. Der authentische Naturstoff hat an entsprechender Position ein Polyen als Substituent (Trienoylfragment). Solche Derivate sind nach dem bisherigen Wissen oxidationsempfindlich. Der Naturstoff ist ebenfalls säurelabil, was bei den erfindungsgemäßen Verbindungen (Zwischenstufen und Endverbindungen) aber nicht der Fall ist.

Es wurde überraschenderweise gefunden, daß bei Verwendung der nachstehend beschriebenen erfindungsgemäßen Scyphostatin-Analoga die Nachteile des natürlich vorkommenden Scyphostatin vermieden werden, d.h., sie weisen, vor allem aufgrund der gegenüber der natürlichen Verbindung einfacheren Struktur, eine erhöhte chemische und metabolische Stabilität auf. Sie eignen sich daher vor allem zur Behandlung von Erkrankungen mit einer inflammatorischen Komponente wie Sepsis, Erkrankungen des rheumatischen Formenkreises, Atherosklerose sowie neurodegenerative Erkrankungen und potentiell Apoptose-vermittelte Erkrankungen wie Morbus Alzheimer, Parkinson, Ischämie, Schlaganfall, Myokardinfarkt und AIDS.

Somit betrifft die vorliegende Erfindung eine Verbindung mit der Formel 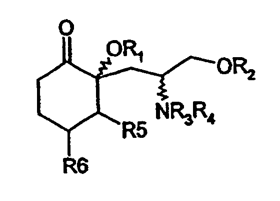 oder 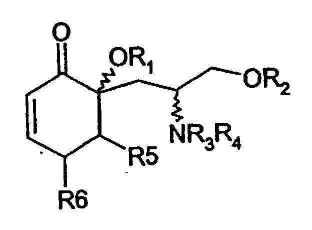 oder 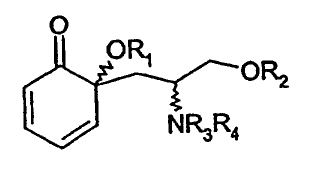 wobei
R1, R2, R3, R5 und R6, die gleich oder verschieden sein können, ein Wasserstoffatom, eine OH-Gruppe, eine SH-Gruppe, ein OAlkyl-, OAryl-, OAcyl-, Sulfonsäure-, Thioalkyl-, Thiophenyl-, Alkyl-, Alkenyl-, Alkinyl-, Aryl- oder Acyl-Rest oder ein Halogenatom sind, und
R4 einen Fettsäurerest, eine C₆-C₂₀ Alkyl-, Alkenyl-, Alkinyl- oder Arylgruppe darstellt, wobei diese Verbindung außerdem dadurch gekennzeichnet ist, daß sie Sphingomylinase (Smase) hemmen kann, oder
ein pharmazeutisch verträgliches Salz davon.

In der vorliegenden Erfindung bedeutet der Ausdruck "Alkylrest" für die Reste R1, R2, R3, R5 und R6 eine geradlinige oder verzweigte C₁-C₁₂-Alkylkette, die z.B. die Methyl-, Ethyl-, n-Propyl-, Isopropyl-, n-Butyl-, Isobutyl, sek-Butyl-, tert-Butyl-, n-Pentyl, Isopentyl-, Neopentyl-, tert-Pentyl-, 2-Methylbutyl-, n-Hexyl-, Isohexyl-, 2-Methylpentyl-, 2,3-Dimethylbutyl-, n-Heptyl-, 2-Methylhexyl-, 2,2-Dimethylpentyl-, 3,3-Dimethylpentyl-, 3-Ethylpentyl-, n-Octyl-, 2,2-Dimethylhexyl-, 3,3-Dimethylhexyl- und 3-Methyl-3-ethylpentylgruppe umfaßt. Bevorzugt sind kurze Alkylketten, wie Methyl-, Ethyl- oder Propyl-. Geeignet sind aber auch Cycloalkylreste mit 3 bis 12 Kohlenstoffatomen. Beispiele hierfür sind eine Cyclopropyl-, Cyclobutyl-, Cyclopentyl-, Cyclohexyl-, Cycloheptyl-, Cyclooctyl-, Cyclononyl- oder Cyclodecylgruppen. Bevorzugt sind Cyclopropyl-, Cyclobutyl-, Cyclopentyl- und Cyclohexyl-. Entsprechendes gilt für die "O-Alkyl-" oder "Thioalkyl"-Gruppe.

Für den Rest R4 weist die Alkylgruppe sechs bis zwanzig C-Atome auf. Bevorzugt sind dabei Gruppen mit mehr als 8 C-Atomen, wie die n-Octylgruppe, n-Nonylgruppe, n-Decylgruppe, 3-Methyl-3-ethylhexylgruppe, n-Undecylgruppe, n-Dodecylgruppe sowie cyclische Alkylreste, wie Cyclooctyl-, Cyclononyl- oder Cyclodecylgruppen.

In der vorliegenden Erfindung bedeutet der Ausdruck "Alkenylrest" für die Reste R1, R2, R3, R5 und R6 gerade oder verzweigte C₂₋₂₀-Alkenylreste. Beispiele hierfür sind Vinyl-, Propenyl-, Isopropenyl-, Allyl-, 2-Methylallyl-, Butenyl- oder Isobutenyl-, Hexenyl- oder Isohexenyl-, Heptenyl- oder Isoheptenyl-, Octenyl- oder Isooctenylgruppen. Bevorzugt sind Vinyl-, Propenyl- und Isopropenyl-. Geeignet sind auch Cycloalkenylreste mit 4 bis 30 Kohlenstoffatomen. Beispiele hierfür sind eine Cyclobutenyl-, Cyclopentenyl- oder Cyclohexenyl-, Cycloheptenyl-, Cxclooctenyl-, Cyclononenyl- oder Cyclodecenylgruppen. Bevorzugt sind Cyclobutenyl-, Cyclopentenyl- oder Cyclohexenyl.

Für den Rest R4 enthält die Alkenylgruppe sechs bis zwanzig C-Atome. Bevorzugt sind Alkenylgruppen mit mehr als 8 C-Atomen, wobei diese sowohl linear, verzweigt oder cyclisch sein können. Analoges gilt für die Alkinylgruppe.

Polyzyklische Alkyl- bzw. Alkenylreste umfassen Norbornan, Adamantan oder Benzvalen.

R1, R2, R3, R4, R5 und R6 können auch ausgewählt sein aus beliebigen mono- oder polycyclischen C₆₋₂₀-Arylresten. Beispiele hierfür sind ein carbocyclischer, monocyclischer Rest, beispielsweise die Phenylgruppe, ein heterocyclischer, monocyclischer Rest, beispielsweise die Gruppen Thienyl, Furyl, Pyranyl, Pyrrolyl, Imidazolyl, Pyrazolyl, Pyridyl, Pyrazinyl, Pyrimidinyl, Pyrazinyl, Thiazolyl, Oxazolyl, Furazannyl, Pyrrolinyl, Imidazolinyl, Pyrazolinyl, Thiazolinyl, Triazolyl, Tetrazolyl, sowie die Positionsisomeren des oder der Heteroatome, die diese Gruppen umfassen können, ein Rest bestehend aus carbocyclischen kondensierten Ringen, beispielsweise die Naphthylgruppe oder die Phenanthrenylgruppe, ein Rest bestehend aus kondensierten heterocyclischen Ringen, beispielsweise Benzofuranyl, Benzothienyl, Benzimidazolyl, Benzothiazolyl. Die Arylreste können unsubsituiert oder substituiert vorliegen. Geeignete Substituenten sind beispielsweise:
- C₁-C₄-Alkyl, wie Methyl, Ethyl-, Propyl, usw.
- substituierte Alkylreste, wie Trifluormethyl-, Trifluorbutyl-, Pentafluorpropyl-, Pentafluorbutyl-, Pentafluorpentyl-, Heptafluorbutyl- oder Nonafluorbutylgruppe oder 2-Chlorethyl-
- Acyloxy, wie Acetoxy oder ein Rest der Formel:

   -O-CO-(CH₂)ₙCO₂H,

   worin n = 1 bis 5,
- Alkoxy, wie Methoxy, Ethoxy, Propyloxy, Isopropyloxy, Butyloxy,
- Alkylthio, wie Methylthio, Ethylthio, Propylthio, Isopropylthio, Butylthio,
- Carbamoyl,
- Alkenyl, wie Vinyl, Propenyl,
- Alkinyl, wie Ethinyl, Propinyl und
- Hydroxy
- Carboxy
- Halogen, wie Fluor, Chlor, Brom, Iod
- CONH₂,
- CONH-Alkyl,
- CON(Alkyl)₂,
- Nitro,
- Amino,
- C₁-C₄-Alkylamino,

In der vorliegenden Erfindung bedeutet der Ausdruck "Acylrest" beispielsweise Acetyl, Propionyl, Butyryl oder Benzoyl. Der Acylrest kann entsprechend auch als O-Acylrest vorliegen.

In der vorliegenden Erfindung bedeutet der Ausdruck "Fettsäurerest" eine aliphatische, gesättigte Carbonsäure mit nahezu ausschließlich unverzweigter Kohlenstoffkette. Bevorzugt enthält der Fettsäurerest 6-30 C-Atome, vorzugsweise ist der Fettsäurerest CO(CH₂)₁₄CH₃.

Der Begriff "Halogenatom" bedeutet Fluor-, Chlor-, Brom- oder Jodatom, wobei Chlor und Brom bevorzugt sind.

In einer bevorzugten Ausführungsform der erfindungsgemäßen Verbindung ist der Sulfonsäurerest OSO₂CH₃.

In einer weiteren bevorzugten Ausführungsform der erfindungsgemäßen Verbindung sind R1, R2, R3, R5 und R6 jeweils ein Wasserstoffatom.

Noch mehr bevorzugt ist eine erfindungsgemäße Verbindung, bei der R4 CO(CH₂)₁₄CH₃ ist, wobei die Verbindung Hexadecansäure-[1-hydroxymethyl-2-(1-hydroxy-2-oxocyclohex-3-enyl)-ethyl]amid (13) am meisten bevorzugt ist.

Der Begriff "pharmazeutisch verträgliches Salz" umfaßt die Salze von anorganischen Säuren, wie z.B. Hydrochlorid, Hydrobromid, Sulfat, Perchlorat, Nitrat und Phosphat, sowie die Salze organischer Säuren, wie z.B. Acetat, Oxalat, Tartrat, Citrat, Succinat, Maleat und Fumarat.

Die erfindungsgemäßen Verbindungen können mit den in den nachfolgenden Beispielen 1 bis 3 beschriebenen Verfahren hergestellt werden. Ihre therapeutische Eignung, d.h. die Hemmung der membrangebundenen neutralen Sphingomylinase (SMase) bzw. die Beeinflussung der intrazellulären Ceramid-Konzentration kann über bekannte Verfahren bestimmt werden; siehe z.B.^{1,2,8,9.}

Die vorliegende Erfindung betrifft auch Arzneimittel, die eine pharmakologisch wirksame Menge einer erfindungsgemäßen Verbindung zusammen mit einem pharmazeutisch verträglichen Träger enthalten. Geeignete Träger und die Formulierung derartiger Arzneimittel sind dem Fachmann bekannt. Zu geeigneten Trägern zählen beispielsweise Phosphat-gepufferte Kochsalzlösungen, Wasser, Emulsionen, beispielsweise Öl/Wasser-Emulsionen, Netzmittel, sterile Lösungen etc. Das erfindungsgemäße Arzneimittel kann in Form einer Injektionslösung, Tablette, Salbe, Suspension, Emulsion, eines Zäpfchens etc. vorliegen. Es kann auch in Form von Depots (Mikrokapseln, Zinksalze, Liposomen etc.) verabreicht werden. Die Art der Verabreichung des Arzneimittels hängt unter anderen davon ab, in welcher Form der Wirkstoff vorliegt, sie kann oral oder parenteral erfolgen. Zu den Verfahren für die parenterale Verabreichung gehören die topische, intra-arterielle, intramuskuläre, intramedulläre, intrathekale, intraventrikuläre, intravenöse, intraperitoneale, transdermale oder transmukosale (nasal, vaginal, rektal, sublingual) Verabreichung. Die Verabreichung kann auch durch Mikroinjektion erfolgen. Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Erkrankung, z.B. AIDS, der Art der Verabreichung etc.

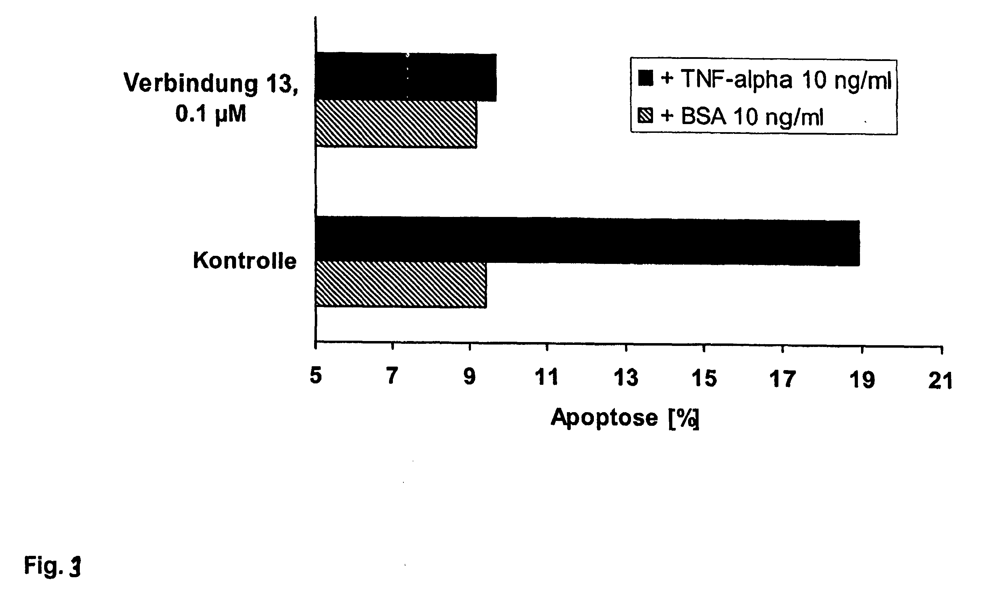

Die vorliegende Erfindung betrifft schließlich auch die Verwendung einer erfindungsgemäßen Verbindung zur Behandlung einer Erkrankung, die mit einer erhöhten Ceramidkonzentration in Zusammenhang steht oder bei der eine Verringerung der Ceramidkonzentration wünschenswert ist. Dabei handelt es sich vorzugsweise um Sepsis, eine Erkrankung des rheumatischen Formenkreises, Atherosklerose, eine neurodegenerative Erkrankung wie z.B. Morbus Alzheimer oder Parkinson, Ischämie, Schlaganfall, Myokardinfarkt oder AIDS. Die antiapototische Aktivität der erfindungsgemäßen Verbindungen ist in Fig. 3 gezeigt.

### Legenden zu den Figuren

Figur 1: Natürlich vorkommendes Scyphostatin **(2)**
Figur 2: Synthese einer Cyclohexenoneinheit
   **(a)** Herstellung der Verbindung (7): a) LDA, THF, -78°C/2h, RT/1h, 84%; b) Et₃N, MsCI, CH₂Cl₂, 0°C, 2h, 98%; c) DBU, CH₂Cl₂, 0°C/1h, RT/12h, 73%; d) Pd/C, H₂, 1 atm, EtOH (96%ig), 30min, 90%.
   **(b)** Synthese der Silyl-Enolether **8** bzw. 9. a) HMDS, TMSI, 0-25°C, THF, 2h, 89%.
   **(c)** Asymmetrische Hydroxylierung. a) AD-Mix-β, NaHCO₃, OsO₄, *tert*-BuOH:H₂O=1:1, 0°C-25°C, 36h, 58%.
   **(d)** Synthese von Scyphostatin-Analoga. a) TFA, CH₂Cl₂, H₂O, RT, 4h, 89%; b) CH₃(CH₂)₁₄COCl, Et₃N, THF, -78°C, 91%.
Figur 3: Antiapototische Aktivität von Verbindung 13 bei humanen Monozyten
   Humanmonozyten werden über 4 Std. mit Tumornekrosefaktor α (TNF α) bzw. mit Albumin (Kontrollen) behandelt. Zusätzlich wird ein Teil der Zellen mit Verbindung **13** vorbehandelt (30 Min., 0,1 *µ*m). Der Prozentsatz apoptotischer Zellen wird in allen Proben durch YOPRO-**1** Färbung¹⁰ ermittelt. Mittelwerte aus 5 Versuchen.

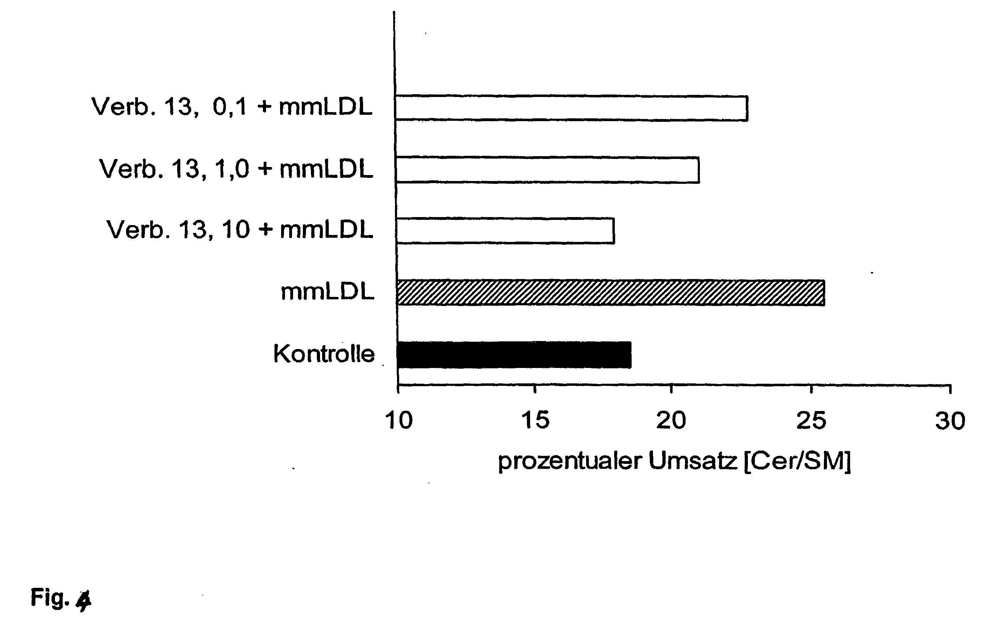

Figur 4: Hemmeffekt von Verbindung **13** (0,1 -10 µm) auf die Sphingomyelinaseaktivität mmLDL-stimulierter Monozyten. Mittelwerte aus 5 Einzelversuchen.

Die nachstehenden Beispiele erläutern die Erfindung.

### Beispiel 1

### Synthese einer Cyclohexenoneinheit

Es wurden vereinfachte strukturelle Analoga der Cyclohexenoneinheit des Scyphostatins hergestellt. Exemplarisch ist die Synthese der Cyclohexenoneinheit mit R1 = R2 = R3 = R5 = R6 = H und R4 = CO(CH₂)₁₄CH₃ beschrieben.

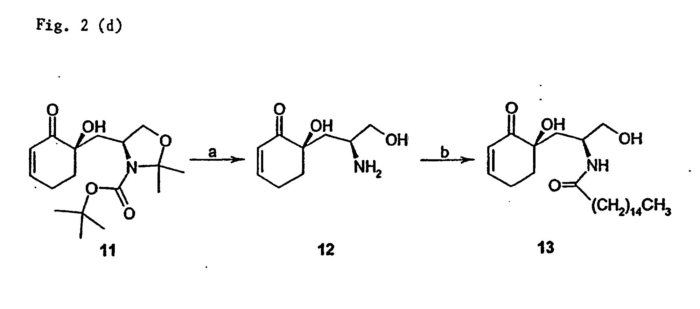

Für die nachfolgende Beschreibung des Verfahrens wird auf Fig. 2 Bezug genommen.

Cyclohexanon bzw. dessen Lithiumenolat und Aldehyd **3** wurden in hohen Ausbeuten zum Alkohol 4 umgesetzt (Figur 2a). Durch Mesitylierung dieses Alkohols und anschließende Eliminierung³ mit DBU wurde das α,β-ungesättigte Keton **6** erhalten. Dieses konnte zum Keton **7** mit Palladium auf Aktivkohle in 96%igem Ethanol hydriert werden.

Bei der Kupplung des Aldehyds **3** an Cyclohexanon wurde am amino-substituierten C-Atom aufgrund der Änderung der Prioritäten die benötigte (S)-Stereochemie erhalten. (Bei von L-Serin abgeleiteten Aldehyd **3b** ausgehend wurde die (R)-konfigurierte Verbindung **4b** erhalten.)

Durch Überführung des substituierten Cyclohexanons **7** in den Silyl-Enolether **8** (Figur 2b) und anschließende asymmetrische Hydroxylierung desselben konnte auch dieses substituierte Cyclohexanon-C-Atom in der (S)-Stereochemie erhalten werden. Analog waren die entsprechenden (R)-Isomere zugänglich, so daß alle Isomere (RS, RR, SR, SS) generiert werden konnten. Hierbei konnte unter Verwendung von Hexamethyldisilazan und Trimethylsilyliodid der thermodynamisch stabilere Enol-Ether **8** gegenüber dem kinetischen Produkt 9 im Verhältnis 85:15 erhalten werden⁴, welches mit Hilfe der ¹H-NMR-Daten ermittelt werden konnte. Die beiden Enolether **8** und **9** wurden chromatographisch getrennt.

Die asymmetrische Hydroxylierung des Silyl-Enolethers **8** zum α-Hydroxy-Keton **10** gelang mit Hilfe der von *Sharpless* beschriebenen Methode⁵ unter Verwendung eines mit Osmium(VIII)oxid angereicherten AD-Mix-β in basischem Milieu (Figur 2c). Die korrekte (S)-Stereochemie konnte durch einen Vergleich mit den für den Naturstoff ermittelten NMR-Daten' nachgewiesen werden. Ein durchgeführtes NOE-Experiment sprach ebenfalls für das Vorliegen der (S)-Stereochemie. Bei Verwendung von AD-Mix (J. Org. Chem. 1992, 57, S. 2768; Aldrichimica Acta 1994, 27, 70) bzw. der darin enthaltenen Einzelkomponenten wurden in der gleichen Größenordnung das (R)-Enantiomere erhalten.

Aus **10** konnte durch Bromierung⁶ das entsprechende αBromderivat erhalten werden, welches durch Dehydrobromierung mit DBU⁷ in das Cyclohexenon **11** überführt werden konnte.

Durch Entschützung der Cyclohexenoneinheit **11** konnte der Aminoalkohol **12** erhalten werden. Bei -78°C ließ sich dieser Aminoalkohol **12** durch Reaktion mit Elektrophilen wie z.B. Säurechloriden in das Scyphostatin-Analoga wie beispielsweise Verbindung **13**, umsetzen (Figur 2d).

Es zeigte sich, daß sich epoxidfreie Scyphostatin-Analoga wie **13** durch erhöhte chemische Stabilität (z.B. Stabilität in sauren Lösungen) auszeichnen und daß sich Analoga mit Substituenten (NHR, R = CO(CH₂)ₙ wie **13** und R = (CH₂)ₙ-R2 (R2 = Aryl, Heteroaryl etc.) auch durch Redoxstabilität (z.B. Oxidation durch Sauerstoff) auszeichnen. Von den Erfindern wurde ermittelt, daß sich Verbindungen, wie **13**, bei pH-Werten von 2 über mehrere Stunden bei 40°C stabil sind.

### Beispiel 2

### Herstellung von 2,2-Dimethyl-4-(2-oxocyclohexylmethyl)-oxazolidin-3-carbonsäure-tert-butylester (7)

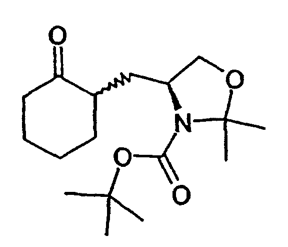

Eine Suspension von 1.39 g 2,2-Dimethyl-4-(2-oxo-cyclohexylidenmethyl]-oxazolidin-3-carbonsäure-tert-butylester **(6)** (4.52 mmol) und 100 mg Palladium auf Kohlenstoff in 50 ml Ethanol (96%ig) wurde 30 Minuten hydriert. Danach wurde der Katalysator über Celite abfiltriert und die Lösung eingeengt. Nach säulenchromatographischer Reinigung (Kieselgel // Ether : n-Hexan = 3 :1) wurde das Produkt als farbloses zähes Öl erhalten (Ausbeute: 1.41 : g = 90%; R_{f}-Wert = 0.72).
^{**1**}**H-NMR** (CDCl₃): *δ* in ppm = 1.43 (s, 6H; C(C*H*_{*3*})_{*2*}), 1.51 (s, 9H; C(CH_{*3*})_{*3*}), 1.60 - 2.38 (m, 11H; C₇*H*_{*11*}O), 3.78 (d, 2H, ³*J* = 7.51 Hz; CHC*H*₂O), 3.98 (m, 1H; CH₂C*H*N). ^{**13**}**C-NMR** (CDCl₃): δ in ppm = 24.65 (CH₂), 26.36 (*C*H₂), 27.26 (C(*C*H₃)₂), 28.37 (C(*C*H₃)₃), 31.14 (CH*C*H₂CH), 33.69 (*C*H₂), 46.11 (CH₂*C*HCO), 55.84 (*C*HN), 67.89 (O*C*H₂CH), 79.82 (*C*(CH₃)₃), 93.58 (*C*(CH₃)₂), 152.54 (COOC(CH₃)₃), 212.13 (*C*O). MS (EI, 60°C), m/z (%) = (C(CH₃)₃), 93.58 (C(CH₃)₂),152.54 (COOC(CH₃)₃), 212.13 (*C*O). MS (EI, 60°C), m/z (%) = 311 (0.61) [M⁺], 253 (1.91) [M⁺-C₄H₉], 196 (34.95) [C₁₀H₁₄NO₃⁺], 180 (7.77) [C₅H₁₄NO₂⁺], 57 (100.0) [C₄H₉⁺]. **IR (Film):** 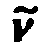 = 2930 (s), 2858 (s), 1711 (s), 1699 (s), 1449 (s), 1388 (s), 1175 (s), 1094 (m), 921 (m) cm⁻¹.

| **Elementaranalyse:** C₁₇H₂₉NO₄ (311.42 g/mol)[%] | | | |
|---|---|---|---|
| ber. | C 65.57 | H 9.38 | N 4.50 |
| gef. | C 65.68 | H 9.27 | N 4.77 |

### Beispiel 3

### Herstellung von Hexadecansäure-[1-hydroxymethyl-2-(1-hydroxy-2-oxo-cyclohex-3-enyl)-ethyl]-amid (13)

Es wurden 145 mg **12** (0.79 mmol) in 50 ml THF gelöst, 1 ml Triethylamin zugefügt und die Lösung auf -78°C gekühlt. Dann wurden 217 mg Palmitinsäurechlorid (0.79 mmol, 0.24 ml) in 5 ml THF langsam zugetropft und die Lösung 15 Minuten bei -78°C gerührt. Die Reaktion wurde mit 25 ml gesättigter Natriumhydrogencarbonatlösung beendet und die Lösung dreimal mit je 30 ml Essigester extrahiert. Die vereinigten organischen Phasen wurden mit 30 ml Wasser gewaschen. Nach dem Trocknen über Magnesiumsulfat wurde im Vakuum bis zur Trockene eingeengt. Es wurde rohes **13** in Form eines sehr schwach gelblichen Feststoffes erhalten. Dieser wurde mit kaltem Äther verrieben und abgesaugt, worauf reines **13** als farbloser Feststoff erhalten wurde (Ausbeute: 302 mg = 91 %).

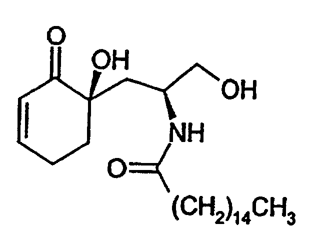

^{**1**}**H-NMR** (CDCl₃): δ in ppm = 0.90 (t, 3H, ³*J* = 6.63 Hz; C*H*₃); 1.24 - 2.21 (m, 34H; 17C*H*₂), 3.27 (b, 2H; 2O*H*), 3.48 (d, 1H, ³*J* = 9.69 Hz; C*H*₂OH), 4.18 (m, 1H, C*H*NH₂), 5.88 (b, 1H; N*H*CO), 6.10 (d, 1H, ³*J*= 9.68 Hz; CHC*H*CO), 7.01 (m, 1H; C*H*CHCO). ^{**13**}**C-NMR** (CDCl₃): δ in ppm =13.89 (*C*H₃), 22.32 - 40.15 (17*C*H₂), 49.41 (*C*HN), 65.21 (HO*C*H₂CH), 78.85 (*C*OH), 128.95 (CO*C*H), 147.33 (*C*HCHCO), 200.88 (*C*O). MS (EI, 58°C), m/z (%) = 424 (0.82) [M⁺], 407 (2.31) [M⁺-OH], 239 (27.88) [C₁₆H₃₁O⁺], 185 (7.46) [M⁺-C₁₆H₃₁O], 94 (8.95) [C₆H₆O⁺]. **IR (Film):** 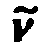 = 3436 (s), 3051 (m), 2977 (s), 2919 (s), 1710 (s), 1635 (s), 1467 (m), 1219 (s), 1122 (s), 1087 (s), 870 (m) cm⁻¹.

| **Elementaranalyse:** C₂₅H₄₅NO₄ (423.63 g/mol)[%] | | | |
|---|---|---|---|
| ber. | C 70.88 | H 10.71 | N 3.31 |
| gef. | C 70.63 | H 10.58 | N 3.53 |

### Beispiel 4

### Sphingomyelinaseaktivität in humanen Monozyten

90 nM BODIPY-FL-C₅ - Sphingomyelin (Molecular Probes) werden in 200 µl Ethanol gelöst, danach in 10 ml PBS injiziert, welches 0,75 mg/ml BSA enthält. Die Lösung wird extensiv gegen PBS dialysiert, das erhaltene Dialysat sterilfiltiert und mit RPMI-Medium 1:1 verdünnt. Mit der Lösung wird ein Monolayer humaner Monozyten über 4 Std. bei 37°C unter 5% CO₂ inkubiert, zweimal mit 5 ml PBS gewaschen, mit neuem Medium versetzt und dann mit den entsprechenden Konzentrationen an Verbindung (13) über 30 Minuten inkubiert. Die vorgehandelten Zellen werden über 4 Std. mit minimal modifiziertem low-density-lipoprotein (mmLDL)¹⁰ behandelt und nach Bligh und Dyer¹¹ extrahiert. Die Enzymaktivität wird über das Verhältnis von BODIPY-FL-C₅ - Sphingomyelin zu BODIPY-FL-C₅ Ceramid bestimmt¹²

Die Ergebnisse des Versuchs sind in Fig. 4 gezeigt.

### Literaturverzeichnis

[1] a) M. Tanaka, F. Nara, K. Suzuki-Konagai, T. Hosoya, T. Ogita, *J. Am. Chem. Soc.,* **1997,** *119,* 7871; b) F. Nara, M. Tanaka, T. Hosoya, K. Suzuki-Konagai, T. Ogita, *J. Antibiot.,* ***1999****, 52, 525.*
[2] S. Saito, N. Tanaka, K. Fujimoto, H. Kogen, *Org. Lett.,* **2000,** 2, 505.
[3] a) M. Suzuki, Y. Oda, N. Hamanaka, R. Noyori, *Heterocycles,* **1990,** *30,* 517; b) D. Meng, S. J. Danishefsky, *Angew. Chem.,* ***1999****, 111,* 1582; c) H. Nagata, K. Ogasawara, *Tetrahedron Lett.,* **1999,** *40,* 6617.
[4] R. D. Miller, D. R. McKean, *Synthesis,* **1979,** 730.
[5] a) K. Morikawa, J. Park, P. G. Andersson, T. Hashiyama, K. B. Sharpless, *J. Am. Chem. Soc.,* **1993,** *115,* 8463; b) H. C. Kolb, M. S. VanNieuwenzhe, K. B. Sharpless, *Chem.Rev.,* **1994,** *94*, 2483.
[6] D. H. R. Barton, J. Boivin, M. Gastiger, J. Morzycki, R. S. Hay-Motherwell, W. B. Motherwell, N. Ozbalik, K. H. Schwartzentruber, *J. Chem. Soc. Perkin, Trans. 1,* **1986,** 947.
[7] J. P. Dulcere, J. Crandall, R. Faure, M. Santelli, V. Agati, M. N. Mihoubi, *J. Org. Chem.,* **1993,** 58, 5702.
[8] M. Harada-Shiba, M. Kinoshita, H. Kamido, K. Shimokado, *J. Biol. Chem.,* 1998, 273, 9681.
[9] S. Kirschnek, F. Paris, M. Weller, H. Grassme, K. Ferlinz, A. Riehle, Z. Fuks, R. Kolesnick, E. Gulbins *J. Biol. Chem.,* **2000,** 275, 27316.
[10] R. Kinscherf, R. Claus, M. Wagner, The FASEB J. **1998,** 12, 461-467
[11] E.G. Bligh und W.J. Dyer, Can. J. Biochem. Physiolol. **1959**, 3, 911-917
[12] R. Kinscherf, R. Claus, H.P. Deigner, FEBS Lett. **1997**, 405, 55-59

## Patentansprüche

1. Verbindung mit der Formel 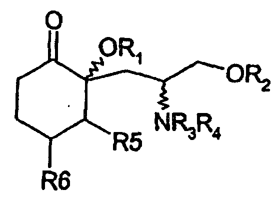 oder 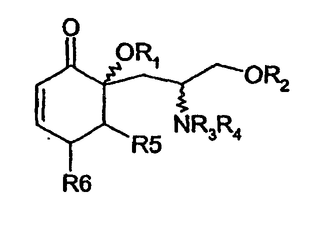 oder 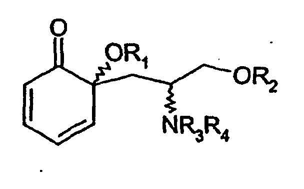 wobei
R1, R2, R3, R5 und R6, die gleich oder verschieden sein können, ein Wasserstoffatom, eine OH-Gruppe, eine SH-Gruppe, ein C₁-C₁₂ OAlkyl-, OAryl-, OAcyl-, Sulfonsäure-C₁-C₁₂ Thioalkyl-, Thiophenyl-, C₁-C₁₂ Alkyl-, C₂-C₂₀ Alkenyl-, C₂-C₂₀ Alkinyl- Aryl- oder Acyl-Rest oder ein Halogenatom sind, und
R4 einen Fettsäurerest, eine C₆ - C₂₀ Alkyl-, C₆-C₂₀ Alkenyl-, C₆-C₂₀ Alkinyl- oder Arylgruppe darstellt,
wobei diese Verbindung außerdem **dadurch gekennzeichnet ist, daß** sie Sphingomylinase (SMase) hemmen kann, oder
ein pharmazeutisch verträgliches Salz davon.

2. Verbindung nach Anspruch 1, wobei der Sulfonsäurerest OSO₂CH₃ ist.

3. Verbindung nach Anspruch 1 oder 2, wobei R1, R2, R3, R5 und R6 ein Wasserstoffatom sind.

4. Verbindung nach einem der Ansprüche 1 bis 3, wobei R4 CO(CH₂)₁₄CH₃ ist.

5. Verbindung nach Anspruch 4, die Hexadecansäure-[1-hydroxymethyl-2-(1-hydroxy-2-oxocyclohex-3-enyl)-ethyl]amid (13) ist.

6. Arzneimittel, umfassend eine pharmakologisch wirksame Menge einer Verbindung nach einem der Ansprüche 1 bis 5 zusammen mit einem pharmazeutisch verträglichen Träger.

7. Verwendung einer Verbindung nach einem der Ansprüche 1 bis 5 zur Herstellung eines Arzneimittels zur Behandlung einer Erkrankung, die mit einer erhöhten Ceramidkonzentration in Zusammenhang steht oder bei der eine Verringerung der Ceramidkonzentration wünschenswert ist.

8. Verwendung nach Anspruch 7, wobei die Erkrankung Sepsis, eine Erkrankung des rheumatischen Formenkreises, Atherosklerose, eine neurodegenerative Erkrankung, Ischämie, Schlaganfall, Myokardinfarkt oder AIDS ist.

## Claims

1. A compound comprising formula 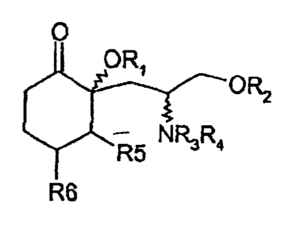 or 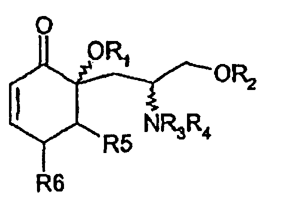 or 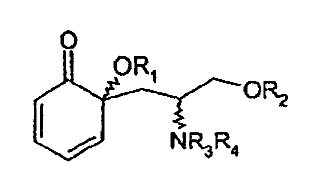 wherein
R1, R2, R3, R5 and R6, which may be equal or different, represent a hydrogen atom, an OH group, an SH group, a C₁-C₁₂-Oalkyl, Oaryl, Oacyl, sulfonic acid, C₁-C₁₂-thioalkyl, thiophenyl, C₁-C₁₂-Alkyl, C₂-C₂₀-Alkenyl , C₂-C₂₀-alkynyl , aryl or acyl group or a halogen atom, and
R4 is a fatty acid residue, a C₆-C₂₀-alkyl, C₆-C₂₀-alkenyl, C₆-C₂₀-alkynyl or aryl group,
said compound being also **characterized in that** it can inhibit sphingomylinase (SMase), or
a pharmaceutically compatible salt thereof.

2. The compound according to claim 1, wherein the sulfonic acid residue is OSO₂CH₃.

3. The compound according to claim 1 or 2, wherein R1, R2, R3, R5 and R6 represent hydrogen atom.

4. The compound according to any of claims 1 to 3, wherein R4 is CO(CH₂)₁₄CH₃.

5. The compound according to claim 4, which is hexadecanoic acid-[1-hydroxymethyl-2-(1-hydroxy-2-oxo-cyclohex-3-enyl)ethyl]amide (13).

6. A pharmaceutical preparation, comprising a pharmacologically active amount of a compound according to any of claims 1 to 5 together with a pharmaceutically compatible carrier.

7. Use of a compound according to any of claims 1 to 5 for the production of a pharmaceutical preparation for treating a disease which is correlated with an increased ceramide concentration or where a reduction of the ceramide concentration is desirable.

8. Use according to claim 7, wherein the disease is sepsis, a disease of the rheumatoid form, atherosclerosis, a neurodegenerative disease, ischemia, apoplexy, myocardial infarction, or AIDS.

## Revendications

1. Composé de formule 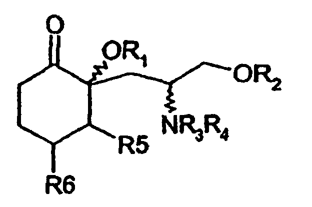 ou 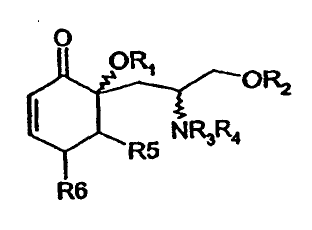 ou 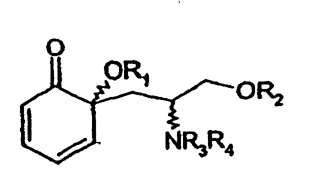 dans laquelle
R1, R2, R3, R5 et R6, qui peuvent être identiques ou différents, représentent un atome d'hydrogène, un groupe OH, un groupe SH, un reste Oalkyle en C₁ à C₁₂, Oaryle ou Oacyle, acide sulfonique, thioalkyle en C₁ à C₁₂, thiophényle, alkyle en C₁ à C₁₂, alcényle en C₂ à C₂₀, alcynyle en C₂ à C₂₀, aryle ou acyle ou un atome d'halogène, et
R4 est un reste d'acide gras, un groupe alkyle en C₆ à C₂₀, alcényle en C₆ à C₂₀, alcynyle en C₆ à C₂₀ ou aryle,
ce composé étant en outre **caractérisé en ce qu'**il peut inhiber la sphingomylinase (SMase), ou
un sel pharmaceutiquement acceptable de ce composé.

2. Composé suivant la revendication 1, dans lequel un reste acide sulfonique est OSO₂CH₃.

3. Composé suivant la revendication 1 ou 2, dans lequel R1, R2, R3, R5 et R6 représentent une atome d'hydrogène.

4. Composé selon l'une des revendications 1 à 3, dans lequel R4 est un groupe CO(CH₂)₁₄CH₃.

5. Composé suivant la revendication 4, qui est le [1-hydroxyméthyl-2- (1-hydroxy-2-oxo-cyclohex-3-ényl)-éthyl] amide d'acide hexadécanoïque (13).

6. Médicament comprenant une quantité pharmacologiquement efficace d'un composé suivant l'une des revendications 1 à 5 conjointement avec un support acceptable du point de vue pharmaceutique.

7. Utilisation d'un composé suivant l'une des revendications 1 à 5 pour la préparation d'un médicament destiné au traitement d'une maladie qui est en rapport avec une concentration élevée en céramide ou pour laquelle une réduction de la concentration en céramide est souhaitable.

8. Utilisation suivant la revendication 7, dans laquelle la maladie est une septicémie, une maladie du domaine des formes rhumatismales, l'athérosclérose, une maladie neurodégénérative, l'ischémie, l'apoplexie cérébrale, l'infarctus du myocarde ou le SIDA.
